# EUROPÄISCHE PATENTANMELDUNG

(11) **EP 2 954 944 A1**
(43) Veröffentlichungstag der Anmeldung: **16.12.2015**
(21) Anmeldenummer: 15170186.9
(22) Anmeldetag: 02.06.2015
(51) Int. Cl.: B01D 61/28, B01D 63/02, B01D 69/08, B01D 71/68

(54) **DIALYSATOR MIT EINEM BÜNDEL AN HOHLFASERN SOWIE VERFAHREN ZUM HERSTELLEN EINER SOLCHEN HOHLFASER**

(30) Priorität: 12.06.2014 DE 102014108230
(71) Anmelder: B. Braun Avitum AG, 34212 Melsungen (DE)
(72) Erfinder: Wolff, Henrik, 37213 Witzenhausen (DE); Strohhöfer, Christof, 34123 Kassel (DE)
(74) Vertreter: Winter, Brandl, Fürniss, Hübner, Röss, Kaiser, Polte - Partnerschaft mbB

(57) **Zusammenfassung**

Die Erfindung betrifft eine Dialysemembran sowie eine Hohlfaser als Vorprodukt sowie ein Verfahren zum Herstellen der Hohlfaser. Die erfindungsgemäße Dialysemembran weist eine Verteilung der Porengrößen auf, die einer Exponentialfunktion und insbesondere einer e-Funktion folgt. Der Kehrwert des Exponentialkoeffizienten (κ) der e-Funktion beträgt wenigstens 30 nm² und insbesondere wenigstens 80 nm². Die Dialysemembran weist wenigstens 50 Poren pro µm² auf, und der Anteil einer freien Durchflussfläche an einer Oberfläche der Dialysemembran beträgt wenigstens 2,5 %.

## Beschreibung

Die Erfindung betrifft einen Dialysator mit einem Bündel an Hohlfasern sowie ein Verfahren zu deren Herstellung.

### Hintergrund der Erfindung

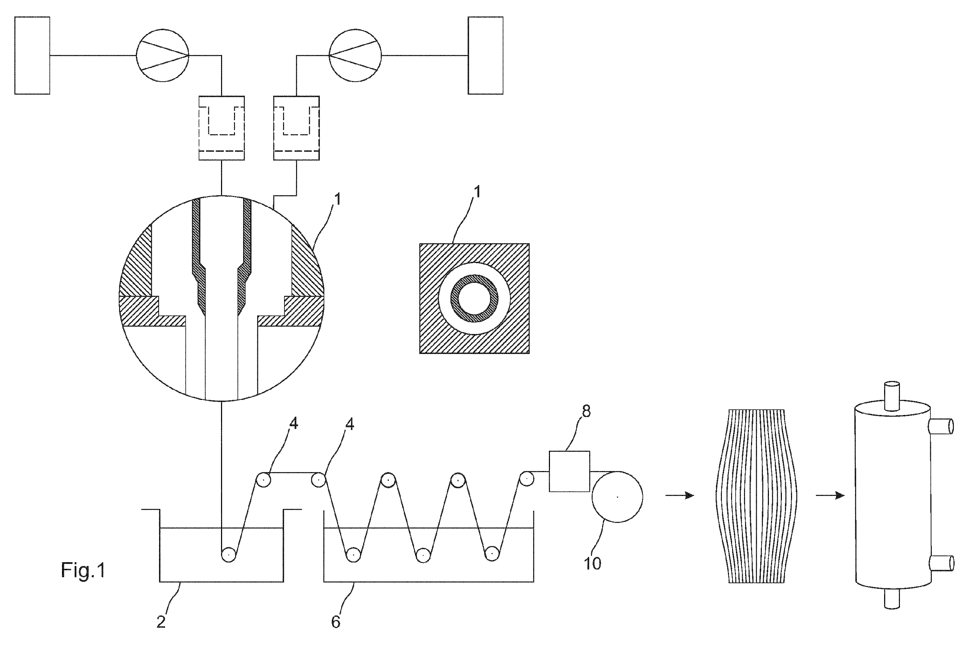

Die Herstellung von Hohlfasern und ihre Weiterverarbeitung zu Membranen für die Dialyse ist grundsätzlich bekannt, beispielsweise aus Uhlenbusch-Körwer, I.; Bonnie-Schorn, E.; Grassmann, A. & Vienken, J. (Ed.) "Understanding Membranes and Dialyzers", Pabst Science Publishers, 2004. Demnach erfolgt die Faserspinnung in sechs grundlegenden Prozessschritten. Dieser Herstellungsprozess ist prinzipiell in der Fig. 1 dargestellt.

Der erste und wichtigste Teilprozess ist demzufolge das Erzeugen einer Hohlfaserform. Dies geschieht in einer Misch- bzw. Ringdüse, in der ein längszentral in die Ringdüse einströmendes Fällmittel in einen Ring (Ringströmung) aus einer Polymerlösung, bspw. Polysulfon, gespritzt wird, um dann aus der Ringdüse axial auszuströmen. Der zweite Teilprozess ist die eigentliche Ausfällung der Polymermembran, die in einem temperierten Wasserbad axial unterhalb der Düse erfolgt. Im nächsten Schritt wird die so entstandene Hohlfaser über Umlenkrollen durch ein Waschbad geleitet, in dem sie von den Resten des Fällprozesses freigespült wird, so dass im nachfolgenden Teilprozess der Trocknung nur noch die reine Hohlfaser behandelt wird. Abschließend werden die Hohlfasern auf eine Spule aufgewickelt, bis die der aufzubauenden Dialysatoroberfläche entsprechende Faseranzahl erreicht ist. Für die Filterherstellung werden aus der auf die Spule aufgewickelten Hohlfaser Faserbündel herausgeschnitten und zum Einbau in ein Dialysatorgehäuse aufbereitet.

### Stand der Technik

Im Stand der Technik wird insbesondere versucht, über ausgewählte Membranparameter die Fluss- und Druckeigenschaften eines Dialysators zu optimieren, so dass eine verbesserte mittelmolekulare Clearance erreicht wird.

Aus der US 5 730 712 A ist allgemein eine Vorrichtung und ein Verfahren zur extrakorporalen Blutbehandlung bekannt. Darin wird ein Strömungswiderstand in einem Dialysator vorgesehen, um den Durchfluss einer Dialysierflüssigkeit durch den Dialysator teilweise zu behindern. Im Ergebnis reicht der Druck in der Dialysierflüssigkeit oberhalb des Strömungswiderstandes aus, um ein nicht-lineares Druckprofil der Dialysierflüssigkeit zu erzeugen. Damit lässt sich beispielsweise mit einem einzigen Dialysator ein großer Wasseranteil aus dem Blut eines Patienten entfernen.

In der EP 1 406 685 A1 (WO 02/098490) wird ein Dialysator beschrieben, der in einem Gehäuse ein Bündel aus mehreren Hohlfasern aufweist. Eine Dialysierflüssigkeit fließt von einem axialen Einlass des Dialysatorgehäuses durch das Faserbündel zu einem axial gegenüberliegenden Auslass des Dialysatorgehäuses, wohingegen Patientenblut nach dem Gegenstromprinzip außerhalb der Hohlfasern axial im Dialysatorgehäuse strömt. Innerhalb des Dialysatorgehäuses bildet das Faserbündel einen mit dem Einlass fluidkommunizierenden Strömungskanal bestehend aus einer Anzahl von Längsrillen im Faserbündel, die sich über den Faserbündelumfang verteilen.

Aus der EP 1 344 542 A1 ist ein Dialysator bekannt, der ein in etwa zylindrisches Gehäuse aufweist, in welchem sich ein Hohlfaserbündel befindet. Das Hohlfaserbündel ist insbesondere in einem Wärmeschrumpfschlauch angeordnet.

Aus der DE 10 2007 009 208 A1 ist eine Hohlfaser, ein Hohlfaserbündel sowie ein Filter daraus und ein Verfahren zur Herstellung derselben bekannt. Die Hohlfasern weisen eine Engstelle als Strömungswiderstand auf.

Aus der US 2007/0119781 A1 sind zudem eine Vorrichtung und ein Verfahren für eine verbesserte Hämodialyse bekannt. Es werden gemäß dieser Druckschrift eine oder mehrere Nanoporen-Röhren als Hämodialysemembran eingesetzt. Diese Röhren können mit einer Nanoporen-Wandstruktur hergestellt werden, bei der der mittlere Porendurchmesser etwa zwischen 5 nm und 10 nm liegt. Die Porengröße einer Dialysemembran wird hierbei im Stand der Technik indirekt durch die Ermittlung von Siebeigenschaften der Membran ermittelt. Dabei wird angenommen, dass die Porengröße einer quasi-Normalverteilung folgt.

Die im Stand der Technik empirisch entwickelten Hohlfasermembranen haben u. a. jedoch die folgenden Nachteile:
1. Die bisher bekannten Hohlfasermembranen sind nicht auf die Anwendung als Dialysator für die extrakorporale Blutbehandlung optimiert. Ihr Porendurchmesser ist kleiner als 6 nm bei Porengrößen unterhalb von 30 nm² für high-flux-Dialysatoren, und der Porendurchmesser ist kleiner als 10 nm bei Porengrößen unterhalb von 80 nm² für high-cut-off-Dialysatoren. Es hat sich gemäß der vorliegenden Erfindung gezeigt, dass diese Werte nicht optimal für die Reinigung mittelmolekularer Substanzen sind.
2. Bei der Beurteilung (Leistungsbewertung) von Hohlfasermembranen werden des Weiteren im Stand der Technik weder die Porendichte noch die freie Durchflussfläche der Membran berücksichtigt.

Vergleicht man Dialysatoren mit Polysulfon-Hohlfasermembranen und sonst gleichen Parametern ergibt sich folgendes Bild:

| | Dialysator 1 | Dialysator 2 | Dialysator 3 |
|---|---|---|---|
| Porengröße nm² | 87 | 164 | 183 |
| Poren / µm² | 62 | 126 | 122 |
| freie Durchflussfläche / % | 1,32 | 2,84 | 3,21 |
| Clearance Cytochrom C / ml/min bei Blutfluss 300 | 100 | 127 | 140 |

Man erkennt hier sehr deutlich, dass die Ausführungsformen gemäß der beispielhaft genannten Dialysatoren 1 bis 3 mit optimierten Membranparametern den Transport von Cytochrom C begünstigen. D.h. die Dialysatorclearance steigt im allgemeinen mit der Zunahme der freien Durchflussfläche an.

In der Dialyse besteht jedoch erfindungsgemäß ein Bedarf an Membranen, die eine Porenanzahl und eine Porengrößenverteilung aufweisen, mit denen sich ein geringer Flusswiderstand für den Transport von Substanzen mit mehr als 500 Da und weniger als 60 kDa erreichen lässt und gleichzeitig ein möglichst hoher Flusswiderstand für den Transport von Substanzen von mehr als 60 kDa sichergestellt ist.

### Kurzbeschreibung der Erfindung

Aufgabe der Erfindung ist es daher, eine Hohlfasermembran sowie ein Verfahren für deren Herstellung einschließlich einer Qualitätskontrolle bereit zu stellen, womit Porenstruktur bzw. Porencharakteristik besser auf die Anforderungen eines Hohlfasermembranfilters für Dialysezwecke (insbesondere extrakorporale Blutbehandlung) abgestimmt ist.

Diese Aufgabe wird gelöst durch einen Dialysator mit Hohlfasern gemäß Anspruch 1 sowie ein Verfahren zum Herstellen einer solchen Hohlfaser nach Anspruch 5. Bevorzugte Ausführungsformen der Erfindung sind Gegenstand der jeweiligen Unteransprüche.

Die Erfindung beruht grundsätzlich auf dem folgenden Ansatz:
Bei der Herstellung einer erfindungsgemäßen Hohlfaser werden erfindungsgemäß ausgewählte Herstellungsparameter beispielsweise das Verhältnis eines hydrophoben Polymers zu einem hydrophilen Polymer vorzugsweise empirisch (bzw. nach dem "trial and error"-Verfahren) derart eingestellt bzw. eingeregelt, dass sich mit dieser Einstellung/Einregelung eine Hohlfaser mit einer erfindungsgemäßen Charakteristika (optimiert für den Einsatz als eine Dialysemembran) ergibt. Die Charakteristika der Dialysemembran sind dabei vorzugsweise
   - die Art der Porengrößenverteilung und/oder
   - die Porengröße und/oder
   - die Porendichte in 1/mm² und/oder
   - die freie Durchflussfläche der Membran in % der Membranfläche.

Die Qualität der Membran kann direkt nach der Erzeugung/dem Spinnen der Hohlfaser auf ihre Eigenschaften geprüft werden, sodass sich auf diese Art die ausgewählten Herstellungsparameter (Einflussgrößen auf die Membraneigenschaften) entsprechend regeln lassen.

Das erfindungsgemäße Verfahren zum Herstellen einer Hohlfaser aus einer Polymerlösung und einem Fällmittel weist demnach zumindest die folgenden Schritte vorzugsweise in der angegebenen Reihenfolge auf:
a) (Vor-) Einstellen von ausgewählten Herstellungsparametern vorzugsweise einer Polymerlösung (Polymermischung),
b) Erzeugen einer Hohlfaserform in einer (Ring-)Düse, in der ein Fällmittel mit einer vorgegebenen Konzentration in einen Ring aus der Polymerlösung gespritzt wird,
c) Ausfällen der Hohlfaser/ Polymermembran in einem temperierten Wasserbad unterhalb der Düse,
d) Leiten der Hohlfaser über Umlenkrollen durch ein Waschbad,
e) Freispülen der Hohlfaser von Resten des Fällprozesses in dem Waschbad,
f) Trocknen der reinen Hohlfaser,
g) Aufwickeln der Hohlfaser auf eine Spule, bis die der Dialysatoroberfläche entsprechende Hohlfaseranzahl erreicht ist,
h) Überprüfen der aktuellen Hohlfasercharakteristik (insbesondere Porengröße und Porendichte sowie vorzugsweise Porengrößenverteilung und freie Durchflussfläche),
i) Vergleichen der aktuellen Hohlfasercharakteristik mit einer (für die extrakorporale Blutbehandlung optimalen) Soll- Hohlfasercharakteristik bzw. einem Hohlfasercharakteristik-Sollbereich und bedarfsweises Nachregeln der ausgewählten Herstellungsparameter, vorzugsweise der Polymerlösungseinstellung, solange/so oft, bis die Soll- Hohlfasercharakteristik angenähert/erreicht ist oder die aktuelle Hohlfasercharakteristik im Hohlfasercharakteristik-Sollbereich liegt.

Vorzugsweise umfasst das Polymer zum Erzeugen der Hohlfaser Polysulfon als hydrophoben Bestandteil. In einer weiteren bevorzugten Ausführungsform des Verfahrens umfasst das Polymer zum Erzeugen der Hohlfaser zusätzlich Polyvinylpyrrolidon (PVP) als hydrophilen Bestandteil.

Desweiteren können Schichten aufgebracht werden, die die Charakteristik der Faser verändern. So kann beispielsweise die Hämokompatibilität der Hohlfaser verbessert werden.

Insbesondere wird das Verhältnis des hydrophoben Polymers zum hydrophilen Polymer auf einen vorgegebenen/analytisch bestimmten Wert eingestellt.

Bei einer weiteren bevorzugten Ausführungsform des Verfahrens wird die Hohlfaser in mehreren Lagen bzw. Wicklungen auf einer Spule aufgewickelt.

Vorzugsweise beträgt die mittels des Verfahrens zu erreichende/anzunähernde Soll-Porengröße auf der Membraninnenseite, dem Teil der Membran, die mit Blut in Kontakt ist, mindestens 30 nm² vorzugsweise mindestens 80 nm². Alternativ liegt die so definierte Soll-Porengröße in einem Bereich von 30-80 nm².

Weiter vorzugsweise beträgt die zu erreichende/anzunähernde Porendichte mindestens 50 Poren/µm².

Weiter vorzugsweise beträgt die freie Durchflussfläche mindestens 2,5 % der gesamten Membranfläche (gemessen "Blutkontakttläche" der Membran)

Vorzugsweise umfasst das Verfahren als weiteren Schritt vor dem Einbau eines aus den Hohlfasern zusammengefasstes Hohlfaserbündels in ein Dialysatorgehäuse (entspricht dem Schritt h)) das Bestimmen der Porengröße der Membran und optional von weiteren Parametern einer Innenseite der Hohlfaser mit einem Elektronenmikroskop.

Insbesondere umfasst das Bestimmen der Porengröße der Membran und optional von weiteren Parametern einer Innenseite der Hohlfaser mit einem Elektronenmikroskop als vorbereitende Schritte:
- Schockgefrieren einer Hohlfaser vorzugsweise in flüssigem Stickstoff,
- Brechen der schockgefrorenen Hohlfaser zum Freilegen der Innenseite der Hohlfaser und
- Ausrichten der Hohlfaser auf einem Objektträger, so dass ein Elektronenstrahl des Elektronenmikroskops auf die Innenseite trifft.

Vorzugsweise umfasst der Verfahrensschritt h) zur Analyse/Überprüfung des erzeugten Produkts die Unterschritte:
- Auftragen der erfassten Porengröße in einem Histogramm und Anpassen einer e-Funktion an die Verteilung der Porengröße,
- Ermitteln einer Anzahl von Poren je µm² und einer freien Durchflussfläche der Membran in Abhängigkeit von der Summe aller Porengrößen und einer Membranoberfläche.

Bevorzugt weist die erfindungsgemäße Hohlfaser/Dialysemembran als ein Merkmal auf, dass die Verteilung der Porengrößen einer Exponentialfunktion und insbesondere einer e-Funktion folgt. Insbesondere beträgt der Kehrwert des Exponentialkoeffizienten (κ) dieser e-Funktion wenigstens 30 nm² und insbesondere wenigstens 80 nm².

Vorzugsweise weist die Hohlfaser/Dialysemembran der vorliegenden Erfindung wenigstens 50 Poren pro µm² auf.

Weiter vorzugsweise beträgt bei der Hohlfaser/Dialysemembran der vorliegenden Erfindung der Anteil einer freien Durchflussfläche an einer Oberfläche der Dialysemembran wenigstens 2,5 %.

Die Erfindung hat u. a. die folgenden Vorteile:
- Es wird eine gesteigerte Effektivität des mittelmolekularen Stofftransports erreicht.
- Desgleichen lässt sich eine optimierte Behandlungsqualität für den Patienten gewährleisten.
- Insgesamt wird durch die neue Porenstruktur auf der Innenseite der Hohlfasermembran die Reinigung mittelmolekularer Substanzen zwischen 500 Da und 60 kDa optimiert.

### Beschreibung erfindungsgemäßer Ausführungsbeispiele

Die vorliegende Erfindung wird nachstehend anhand eines bevorzugten Ausführungsbeispiels unter Bezugnahme auf die begleitenden Figuren näher erläutert.
Fig. 1 zeigt den prinzipiellen/funktionalen Aufbau einer Einrichtung zur Herstellung von für den Einbau in Dialysatoren geeigneten Hohlfasern,
Fig. 2 zeigt die Aufnahme einer erfindungsgemäßen Hohlfaser/Membran unter einem Elektronenmikroskop,
Fig. 3 zeigt die rechnerische Aufbereitung der Aufnahme gemäß der Fig. 3 zur Darstellung der in der Membran ausgebildeten Poren sowie der Porenverteilung und
Fig. 4 zeigt ein aus der Darstellung gemäß der Fig. 2 erstelltes Histogramm mit der Porengrößenverteilung .

Das Verfahren zum Herstellen der erfindungsgemäßen Hohlfaser aus einer Polymerlösung und einem Fällmittel lässt sich in die folgenden Schritte aufteilen.

Zunächst wird eine Hohlfaserform in einer Düse 1 erzeugt, indem in der Düse 1 das Fällmittel mit einer vorgegebenen Konzentration in einen Ring aus einer ausgewählten (voreingestellten) Polymerlösung gespritzt wird. Anschließend wird die Hohlfaser/Polymermembran in einem temperierten Wasserbad 2 unter (stromab) der Düse 1 ausgefällt. Über Umlenkrollen 4 wird die Hohlfaser durch ein Waschbad 6 geleitet, wo sie von Rückständen aus den vorangehenden Prozessschritten gereinigt wird. Daran schließt sich ein Freispülvorgang an, bei dem die Hohlfaser von Resten des Fällprozesses in dem Waschbad befreit wird. Schließlich wird die nun gesäuberte Hohlfaser in einem Trockner 8 getrocknet und auf eine Spule 10 aufgewickelt. Der Aufwickelvorgang wird ausgeführt, bis die der Dialysatoroberfläche entsprechende Hohlfaseranzahl erreicht ist. In soweit entspricht das Herstellungsverfahren dem Stand der Technik.

Als Polymer zum Erzeugen der Hohlfaser wird vorzugsweise Polysulfon als hydrophober Bestandteil eingesetzt, und als hydrophiler Bestandteil des Polymers wird vorzugsweise Polyvinylpyrrolidon (PVP) eingesetzt. Das Verhältnis der beiden Komponenten wird zunächst auf einen vorgegebenen Wert (Erfahrungswert) eingestellt.

Aus der derart hergestellten Hohlfaser wird mit den folgenden Schritten eine Dialysemembran erzeugt:
Aus der auf die Spule 10 aufgewickelten Hohlfaser wird ein Hohlfaserbündel (Nicht weiter dargestellt, da aus dem Stand der Technik bekannt) herausgeschnitten. Das herausgeschnittene Hohlfaserbündel wird in ein Dialysatorgehäuse (ebenfalls aus dem Stand der Technik bekannt und daher nicht weiter dargestellt) eingebaut und dient als Dialysefilter.

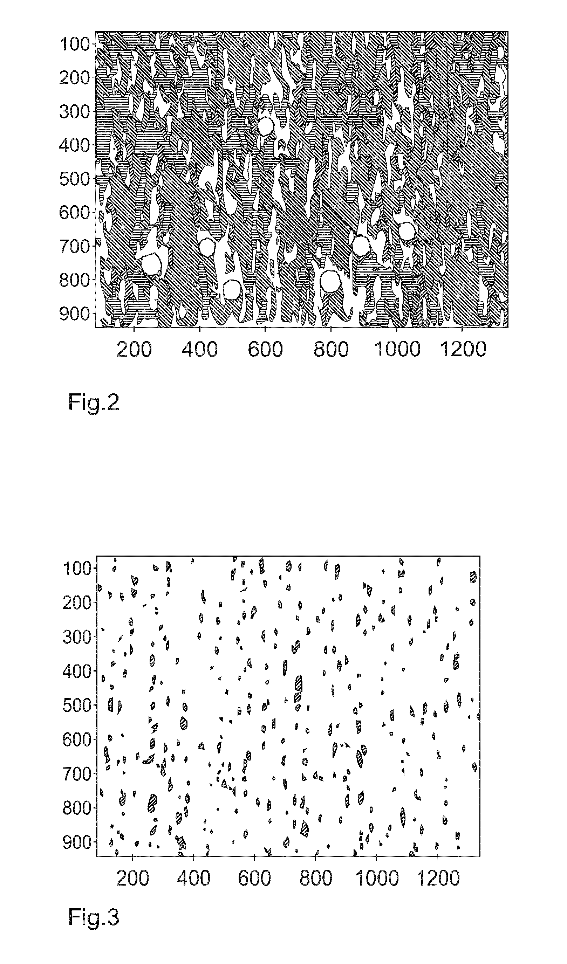

Zur Sicherstellung der Eigenschaften/Charakteristika der Dialysemembran werden einzelne Parameter, bevorzugt die Porengröße, bzw. die Porengrößenverteilung, der Hohlfaser/Membran bestimmt und optional weitere Parameter der Innenseite der Hohlfaser mit einem Elektronenmikroskop untersucht. Hierzu wird auf die begleitenden Fig. 2 bis 4 verwiesen.

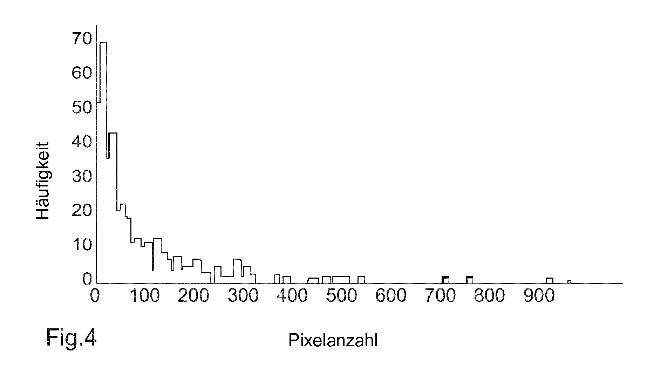

Demzufolge werden die Hohlfasern und einem Elektronenmikroskop bildtechnisch erfasst. wodurch sich eine Oberflächenabbildung der Hohlfaser beispielsweise gemäß der Fig. 2 ergibt. Diese Abbildung wird dann einer rechnerischen Aufbereitungsprozedur unterzogen, aus der sich eine Schwarz-Weiß-Darstellung in etwa gemäß der Fig. 3 ergibt, in der nur die Poren dargestellt werden. In dieser Schwarz-Weiß-Darstellung können dann beispielsweise über Pixelanzahlen bzw. über den Maßstab der Aufnahme die Porengröße, sowie deren (Poren-) Anzahl bestimmt werden. Daraus lässt sich ein Histogramm gemäß der Fig. 4 erstellen mit der Porengrößenverteilung indem beispielsweise die "Häufigkeit" gegen "Pixelanzahl" aufgetragen wird, wobei alternativ die "Pixelanzahl" natürlich auch durch die Fläche (nm²) ersetzt bzw. entsprechend umgerechnet werden könnte.

Diese Parameter der Dialysemembran zu verifizieren ist im Stand der Technik bisher nicht oder nur eingeschränkt/indirekt vorgenommen worden. Daher wurden im Stand der Technik auch nur entsprechende Annahmen über die Verteilung der Poren in der Membran gemacht. Insbesondere wurde vereinfacht von einer quasi-Normalverteilung für die Porengrößen ausgegangen, da deren unmittelbarer Einfluss auf die Eignung der Hohlfaser für bestimmte Zwecke (extrakorporale Blutbehandlung) nicht erkannt bzw. unterschätzt wurde. Die Porengröße wurde daher auch nur aus Rückschlüssen aus den Siebeigenschaften der Membran abgeschätzt. Sie wurde nicht direkt gemessen.

Eine Charakterisierung der Membran erfolgte im Stand der Technik viel mehr über die Siebeigenschaften der Membran, also über die Größe der Moleküle, die die Membran passieren können.

Erfindungsgemäß hingegen wird zur qualitativen Beurteilung der erzeugten Membran und damit zur Einstellung des Hohlfaser-Herstellungsprozesses die Porengröße der Hohlfaser/Membran vorzugsweise mit elektronenmikroskopischer Messtechnik untersucht. Mit dem Elektronenmikroskop können die charakteristischen Parameter der Faserinnenseiten ermittelt werden. Die direkte elektronenmikroskopische Visualisierung der Poren liegt dabei am Auflösungslimit der derzeitigen Technologie und liefert gesicherte Messergebnisse als Grundlage für die Charakteristik-/Qualitätsbestimmung der Hohlfaser und ggf. für die Nachregelung der Herstellungsparameter.

Zur Probenpräparation werden einzelne Fasern in flüssigem Stickstoff schockgefroren. Anschließend werden die Fasern gebrochen, um Aufnahmen von der Oberfläche der Faserinnenseite anzufertigen. Schließlich wird die Faser auf einem Objektträger ausgerichtet, so dass der Elektronenstrahl auf die Innenseite der Fasern gerichtet werden kann.

Vorzugsweise werden die Rasterelektronenmikroskopieaufnahmen mit einem Rasterelektronenmikroskop aufgenommen, das beispielsweise bei einer Beschleunigungsspannung von 3 kV betrieben wird und eine 50.000-fache Vergrößerung ermöglicht. Die Probenpräparation erfolgt wie oben erwähnt durch Schockgefrieren einzelner Hohlfasern in flüssigem Stickstoff, Brechen der Hohlfasern zum Freilegen der Innenseite der Hohlfaser, so dass Aufnahmen von der Oberfläche der Faserinnenseite angefertigt werden können, und Ausrichten der Hohlfaser auf einem Objektträger, so dass ein Elektronenstrahl des Elektronenmikroskops auf die Innenseite trifft.

Die Verteilung der Porengröße wird in einem Histogramm dargestellt. Die Porengrößenverteilung in dem Histogramm lässt sich erfindungsgemäß wiederum mit einer e-Funktion beschreiben, wobei eine charakteristische Größe der e-Funktion verwendet wird, der Kehrwert des Exponentialkoeffizienten (κ). Dieser lässt sich einfach ermitteln, da an dieser Stelle eine e-Funktion des Typs f(x)= A*e^(-(κ)*x) den Wert {(1/e) * A} annimmt (mit A = Maximalwert).

Ferner lässt sich die Anzahl von Poren je µm² ermitteln und die freie Durchflussfläche der Membran in Abhängigkeit von der Summe aller Porengrößen und einer Membranoberfläche bestimmen. Die Porendichte ist die Anzahl der Poren je µm², und die freie Durchflussfläche setzt die Summe aller Porengrößen ins Verhältnis zur vermessenen Oberfläche.

Die derart hergestellte Hohlfaser/Dialysemembran weist bei der Verteilung der Porengrößen eine Exponentialfunktion und insbesondere eine e-Funktion auf. Als besonders geeignete Hohlfaser/Dialysemembran werden diejenigen ausgewählt, bei denen der Kehrwert des Exponentialkoeffizienten (κ) der e-Funktion wenigstens 30 nm² und insbesondere wenigstens 80 nm² beträgt. Als Porendichte wird insbesondere wenigstens 50 Poren pro µm² angestrebt, und der Anteil der freien Durchflussfläche an der gesamten Oberfläche der Dialysemembran beträgt wenigstens 2,5 %.

Zusammenfassend weist die erfindungsgemäße Membran folgende Eigenschaften auf:
I. Die Porengrößenverteilung folgt einer Exponentialfunktion.
II. Der Kehrwert des Exponentialkoeffizienten (κ) beträgt mindestens 30 nm² im Falle von high-flux-Dialysatoren und vorzugsweise mindestens 80 nm².
III. Die Porendichte beträgt mindestens 50 Poren/µm².
IV. Die freie Durchflussfläche beträgt mindesten 2,5 %.

Damit betrifft die Erfindung eine Dialysemembran sowie eine Hohlfaser als Vorprodukt sowie ein Verfahren zum Herstellen der Hohlfaser. Die erfindungsgemäße Hohlfaser/Dialysemembran weist eine Verteilung der Porengrößen auf, die einer Exponentialfunktion und insbesondere einer e-Funktion folgt. Der Kehrwert des Exponentialkoeffizienten (κ) der e-Funktion beträgt wenigstens 30 nm² und insbesondere wenigstens 80 nm². Die Hohlfaser/Dialysemembran weist wenigstens 50 Poren pro µm² auf, und der Anteil einer freien Durchflussfläche an einer Oberfläche der Hohlfaser/Dialysemembran beträgt wenigstens 2,5 %.

## Patentansprüche

1. Dialysator zur extrakorporalen Blutbehandlung eines Patienten mit einem Dialysatorgehäuse, in dem ein Bündel aus Hohlfasern untergebracht ist, von denen jede Hohlfaser Poren aufweist für den Durchlass von maximal mittelmolekularen Substanzen, **dadurch gekennzeichnet, dass** die Verteilung der Porengrößen an der Hohlfaserinnenseite einer Exponentialfunktion und insbesondere einer e-Funktion folgt, wobei vorzugsweise der Kehrwert des Exponentialkoeffizienten (κ) der vorzugsweise e-Funktion wenigstens 30 nm² und vorzugsweise wenigstens 80 nm² beträgt.

2. Dialysator nach dem Oberbegriff des Anspruchs 1, **dadurch gekennzeichnet, dass** die Porengröße wenigstens 30 nm² und vorzugsweise wenigstens 80 nm² beträgt.

3. Dialysator nach einem der Ansprüche 1 oder 2, **dadurch gekennzeichnet, dass** die Hohlfaser wenigstens 50 Poren pro µm² aufweist.

4. Dialysator nach einem der Ansprüche 1 bis 3, **dadurch gekennzeichnet, dass** ein Anteil einer freien Durchflussfläche an der inneren Oberfläche bzw. Blutkontaktfläche der Hohlfaser wenigstens 2,5 % beträgt.

5. Verfahren zum Herstellen einer Hohlfaser aus einer Polymerlösung und einem Fällmittel mit den Schritten:
a) Voreinstellen von ausgewählten Herstellungsparametern vorzugsweise der Polymerlösung,
b) Erzeugen einer Hohlfaserform in einer Düse, in der das Fällmittel mit einer vorgegebenen Konzentration in einen Ring aus der Polymerlösung gespritzt wird,
c) Ausfällen der Hohlfaser in einem temperierten Wasserbad,
d) Leiten der Hohlfaser vorzugsweise über Umlenkrollen durch ein Waschbad,
e) Freispülen der Hohlfaser von Resten des Fällprozesses in dem Waschbad,
f) Trocknen der reinen Hohlfaser,
g) Aufwickeln der Hohlfaser auf eine Spule,
h) Überprüfen der aktuellen Hohlfasercharakteristik insbesondere Porengröße und Porendichte sowie vorzugsweise Porengrößenverteilung und freie Durchflussfläche,
i) Vergleichen der aktuellen Hohlfasercharakteristik mit einer für die extrakorporale Blutbehandlung optimalen Soll- Hohlfasercharakteristik bzw. einem Hohlfasercharakteristik-Sollbereich sowie bedarfsweises Nachregeln der ausgewählten Herstellungsparameter, vorzugsweise der Polymerlösungseinstellung, solange/so oft, bis die Soll-Hohlfasercharakteristik angenähert/erreicht ist oder die aktuelle Hohlfasercharakteristik im Hohlfasercharakteristik-Sollbereich liegt.

6. Verfahren nach Anspruch 5, **dadurch gekennzeichnet, dass** das Polymer zum Erzeugen der Hohlfaser Polysulfon als hydrophoben Bestandteil umfasst und vorzugsweise das Polymer zum Erzeugen der Hohlfaser Polyvinylpyrrolidon (PVP) als hydrophilen Bestandteil umfasst.

7. Verfahren nach Anspruch 6, **dadurch gekennzeichnet, dass** das Verhältnis des hydrophoben Polymers zum hydrophilen Polymer auf einen vorgegebenen und/oder analytisch bestimmten Wert als Herstellungsparameter eingestellt wird.

8. Verfahren nach einem der vorangehenden Ansprüche 5 bis 7, **gekennzeichnet durch** den Schritt: Aufwickeln der Hohlfaser in mehreren Lagen auf der Spule.

9. Verfahren nach einem der vorstehenden Ansprüche 5 bis 8, **gekennzeichnet durch** den Schritt: Bestimmen der Porengröße der Hohlfaser und optional von weiteren Parametern einer Innenseite der Hohlfaser vorzugsweise mit einem Elektronenmikroskop.

10. Verfahren nach Anspruch 9, **dadurch gekennzeichnet, dass** das Bestimmen der Porengröße der Membran und optional von Parametern einer Innenseite der Hohlfaser mit einem Elektronenmikroskop umfasst: Schockgefrieren einer Hohlfaser in flüssigem Stickstoff, Brechen der Hohlfaser zum Freilegen der Innenseite der Hohlfaser, Ausrichten der Hohlfaser auf einem Objektträger, so dass ein Elektronenstrahl des Elektronenmikroskops auf die Innenseite trifft.

11. Verfahren nach einem der Ansprüche 5 bis 10, **gekennzeichnet durch** die Schritte: Auftragen der Porengröße in einem Histogramm und Anpassen einer Exponentialfunktion, vorzugsweise e-Funktion an die Verteilung der Porengröße, Ermitteln einer Anzahl von Poren je µm² und einer freien Durchflussfläche der Membraninnenseite in Abhängigkeit von der Summe aller Porengrößen und einer Membranoberfläche.
